# EUROPEAN PATENT SPECIFICATION

(11) **EP 1 374 787 B1**
(45) Date of publication and mention of the grant of the patent: **27.06.2007**
(21) Application number: 01943980.1
(22) Date of filing: 26.02.2001
(51) Int. Cl.: A61B 17/66, A61B 17/64

(54) **FIXATOR FOR HAND AND FOOT BONES**
FIXATEUR FÜR HAND- UND FUSSKNOCHEN
DISPOSITIF DE FIXATION POUR DES OS DE LA MAIN ET DU PIED

(43) Date of publication of application: 02.01.2004
(73) Proprietor: Fed. State Institution of Science Russian Ilizarov Scient. Ctr. Restorative Traumatology & Orthopaed. Federal Agency of Health & Social Development, Kurgan 640005 (RU)
(72) Inventor: SHEVTSOV, Vladimir Ivanovich, Kurgan, 640020 (RU); ISMAILOV, Guseinali Rustam Ogly, Kurgan, 640020 (RU); BURLAKOV, Eduard Valentovich, Kurgan, 640020 (RU); NEMKOV, Vitaly Anatolievich, Kurgan, 640001 (RU)
(74) Representative: Hano, Christian
(86) International application number: PCT/RU2001/000084
(87) International publication number: WO 2002/067790

(56) References cited:
- EP-A- 0 700 664
- EP-A2- 0 913 128
- RU-U1- 2 186
- SU-A- 1 708 319
- SU-A1- 1 708 319
- SU-A1- 1 715 333
- US-A- 4 978 348
- US-A- 5 921 985
- US-A- 5 961 515

## Description

### Field of the Invention

The invention belongs to the sphere of medical equipment used in orthopaedics and traumatology, particularly to surgical instruments assigned for osteosynthesis namely for external fixation devices located along the bone and with the possibility of biological tissue lengthening.

### Description of the Prior Art

External mini-fixators are used for management of bone lesions (Klinik für orthopädische Chirurgie der Universität Bern, Schwi, Roiand P.Jakod "Der Kleine Fixateur externe").

The fixator contains interconnected rods and wires with threaded ends placed in wire-clamps. The wires are positioned and fixed at 40-60 degrees angle.

The described device allows gradual reposition and then fixation of the damaged bones.

The wires are fixed in the plane parallel to the longitudinal axis of the rod bearing wire-clamps each of which has one wire. It leads to increase of longitudinal size of the fixator. Besides, resorption can occur during treatment and it leads to wire loosening.

A device for orthopaedic axial fixation (FR, A, 255938) is known. The device contains a metal rod having the shape of parallelepiped bearing two wire-clamps. Parts of the wire-clamp are fixed with the help of a screw. One of the clamps is positioned with the possibility of longitudinal transfer with the help of threaded spindle.

The described device allows fixation of tubular bones and longitudinal transfer of bone fragments. However sliding of half-wires out of bone during load application can happen when there are parallel half-wires. Besides, slots for wires in clamps are made at a certain distance from each other and they determine wire position in a particular place that is not always permissible for a certain bone part.

An orthopaedic apparatus assigned for external axial fixation of bone or bone fragments with wide range of control is used for management of bone lesions (GB, A, 2168255).

The apparatus has central frame consisting of three or more parts, they can move relatively at the expense of telescopic sliding without the possibility of relative rotation. Parts of the frame have blocking devices, which can be activated separately for each pair of parts.

Besides, there is a power cylinder equipped with at least three elements with possibility of screwing the latter into each other and transfer along the common axis when rotating one of the elements. The elements are positioned at the end of the cylinder and are provided with pins, which can go into eccentric cavities of the cylindrical frame.

The described apparatus can regulate position of the elements with pins relatively to the central frame and each other. However the pins are positioned in the openings of the element at a certain distance from each other and also predetermine inconvenience of application because of absence of free choice for rod position in a bone. The orthopaedic apparatus assigned for external axial fixation has no possibility to regulate the angle of wire position in the element. Besides, wire insertion is done in dorsal-plantar direction and it leads to damage of hand muscles-flexors.

Dynamometric external fixator known from GB, A, 2146533, and U.S., 4,628,919 is used for management of hand joint lesions. It has two oblong supports with fixation wires in each of them. Both the supports are connected by universal hinge, which has elements to regulate the degree of transfer of the distal support relatively to the proximal support. The proximal support has several openings for introduction of fixation wires and appropriate threaded locking elements. The distal support has a piston for length regulation. The adjusting screw, scrollable reciprocally in the slot of the distal support, is used to regulate the degree of piston advancing. Dynamometric external fixator enables fixation of the joint of two adjacent bones along the lateral wrist surface.

The described fixator has the possibility to regulate the degree of transfer of the distal support relative to the proximal one and to determine the degree of transfer. Besides, wires of the distal support can be transferred relative to wires of the proximal support. However, openings for wires in the supports of the fixator are made at a fixed distance from each other. Besides, application of dynamometric external fixator for adjacent phalanges and simultaneous placement for adjacent metacarpal bones and phalanges is made difficult.

US 5, 961,515 relates to a pin-to-bar construct for an external fixation of the type including a spool component for attachment to a bar, and an intermediate component for fixed attachment to the spool component when the intermediate component has an aperture for joining one of the pins. Said construct for an external fixation makes possible to fix bone fragments in bone fractures but doesn't allow their gradual longitudinal transport with regard to the bar to perform compression or distraction between the bone fragments due to the attachment of the spool components with pins to the bar. Also, usage of direct pins in bone fixation increases transverse construct dimension and doesn't allow simultaneous fixation of several adjacent bones of hand or foot.

From EP 0 700 664 A1 is known a connecting junction for components of external fixation including clamps with slots for a rod or a pin, appliance for compression passing through the clamps and elastic means to maintain temporarily the pins and rods prior to the blockage of the junction. Said connecting junction is provided for fastening of one pin to the rod under angle in the plane parallel to its axis. This direction of the rods is not acceptable for the hand and foot bones fixation and the absence of rods transport possibility with regards to the pins doesn't allow to perform compression or distraction between the bone fragments.

US 5,921,985 relates to an orthopedic fixation device using circular rod stock, threaded fixation pins and sectional clamps for rigid fixation of fixation pins to the circular rod stock, wherein said clamps having dual sections with semicircular cutouts and a bolt device including a hole and a washer with semicircular divot for fixation pin. Said construction has no capacity for graduated manipulations with fixation pins during treatment. Separate fixation of each fixation pin increases a distance between them and closes out the fixation of short bone fragments.

US 1,708,319 discloses a compression-distraction device including arch-shaped support and attached distractors with wire-clamps having slots, magnet inlays, guiding skews and grooves. However, orientation of washers in the wire-clamps relative to wires using the grooves and guiding skews complicates frame assembly. Fixation of wires in the washers' slots causes rotation of washers with wires and translation of fixing bone fragment. Temporary wire fixation with magnet inlays considerably increases the price of a device. From US 4,978,384 is known a compression and distraction apparatus for osteosynthesis which is more advanced because it allows distraction. This apparatus contains a threaded rod. At least two wire-fixation devices with the possibility of transfer are mounted on the rod. Each of the wire-fixation devices is made as a bolt and a set of washers is mounted on it. Each of the wire-fixation devices is positioned with the possibility of longitudinal transport on the threaded rod.

However the named construction makes it difficult to keep stable position of bone fragments because there is a possibility of turning of the fixation elements when fixing wire ends. This construction doesn't allow fixation of small osteotomized fragments. Besides, the named device doesn't have details of different sizes to use them considering age criteria.

### Summary of the Invention

The object of the present invention is to provide a fixator for hand and foot bones preventing translation of bone fragments in their fixation with widened functional possibilities to get stable osteosynthesis of short bones and simple mounting of the construction during surgical intervention, manipulation with bone fragments of short bones during treatment. It leads to increase of treatment efficiency.

This task can be solved the following way: hand and foot bone fixator contains at least two supporting elements each of which includes settling bearer made as a rectangular prism having settling cylinders and longitudinal through adjusting opening on both front surfaces; clamping element made as a polyhedral prism having base and through longitudinal opening, the mentioned base is made as a cylinder cut off on opposite sides and it has open longitudinal rectangular slot, it includes through adjusting opening, axis of which is parallel to the mentioned planes of cutting off; two fastening nuts; rod, having each of the supporting elements mounted by means of two regulating nuts with the possibility of transport; wires: some ends of them go through bone and the other ends are attached to the supporting elements.

Making an opening in one of the lateral edges of each settling bearer is worthwhile for holding the settling bearer in the desired position. Axis of the opening should be perpendicular to the axis of the mentioned longitudinal adjusting through opening; locking element should be placed in the longitudinal adjusting through opening.

Besides, the opening in each of the mentioned settling bearer can be done in one the side planes of the prism. The axis of the opening is positioned perpendicular to the axis of the mentioned longitudinal adjusting through opening.

As a variant of invention, the locking element can be made as a screw with front surface perpendicular to its longitudinal axis to make mounting of the locking element convenient.

Ends of the mentioned wires are positioned at an angle to each other to prevent damage of the hand tendon apparatus and to make the size of the supporting element smaller during fixation of short bones.

Positioning an attached end of each of the mentioned wires parallel to another one and a plunged into bone end of each of the mentioned wires at an angle is worthwhile to make centering of supporting elements easier and to improve fixation rigidity.

To widen the range of application for hand and foot bone fixator it should be supplied at least two supporting elements. The first of them includes an settling bearer made as a bushing having a head with an opening axis of which is perpendicular to the axis of the bushing; locking element; fixating element made as a prism having an open rectangular slot and through longitudinal adjusting opening; the additional fixating element made as a prism having an open slot and a through longitudinal opening; fixing element; two regulating elements; wires, some ends of which are attached in the mentioned supporting element. And the second supporting element includes settling bearer made as an arch; connecting element made as a bracket and attached to the mentioned settling bearer; wire-fixation devices placed at the ends of the latter with ends of at least two wires attached by them. The first mentioned support element is placed with the possibility to be transported by means of two regulating elements on the rod having longitudinal flat spot; one of the ends of the mentioned rod is connected with the possibility of angular transport with the mentioned second supporting element.

For fixation of short osteotomized fragments of hand and foot bones it is worthwhile to supply the fixator of hand and foot bones with at least two supporting elements. The first element includes settling bearer made as a bushing with head and having threading on internal and external surfaces of the mentioned bushing. The mentioned head can be made as a plate and the clamping element - as a prism with the base and a through longitudinal opening; additional clamping element is made as a prism with an open slot and a through longitudinal adjusting opening; besides, the mentioned head should have a fixing element. The second supporting element can include: settling bearer made as a bushing with head, its internal surface is made smooth and there is a threading on the external surface. The clamping element can be made as a prism with base and a through longitudinal adjusting opening; additional clamping element - as a prism having open slot and a through longitudinal adjusting opening and having a link, clamping element. Both the supporting elements are interconnected by rod. One end of the rod has a threading. A connecting plate and a fixing element are placed at the mentioned end. The second supporting element is positioned on the smooth end of the mentioned rod. For this purpose the mentioned connecting plate and the link are supplied with through openings by means of which they are connected by an additional rod with the help of two pairs of additional regulating nuts.

As a variant, the rod and the additional rod are supplied with a hinge and the mentioned plate is made as a link; both the links are positioned respectively on the first and the second supporting elements and the mentioned articulated joins are positioned respectively between the first and the second supporting elements. All this is done to have the possibility of angular transport.

The patented invention is explained by detailed description, example of realization and accompanying schemes, in which:
FIG. 1 is a general view of the hand and foot bone fixator, fulfilled according to the invention;
FIG. 2 is a supporting element of the fixator according to the invention, disassembled;
FIG. 3 is a variant of wire attachment in the supporting element; ends of the wires are positioned at an angle to each other;
FIG. 4 is a variant of settling bearer according to the invention with an opening for the locking element made in one of its lateral sides;
FIG. 5 is a side view of the supporting element; the same as the fig.4; the attached wire-ends are parallel to each other;
FIG.6 is a view of the fixator according to the invention; one of the supporting elements is made as an arch;
FIG. 7 is the first supporting element of the fixator, disassembled; the same as fig. 6.
FIG. 8 is a variant of the fixator, which has two supporting elements interconnected with the help of an additional rod according to the invention;
FIG. 9 is a possible variant to connect the rod and the additional rod according to the invention with the help of articulated joins.

### Description of Implementation of the Invention

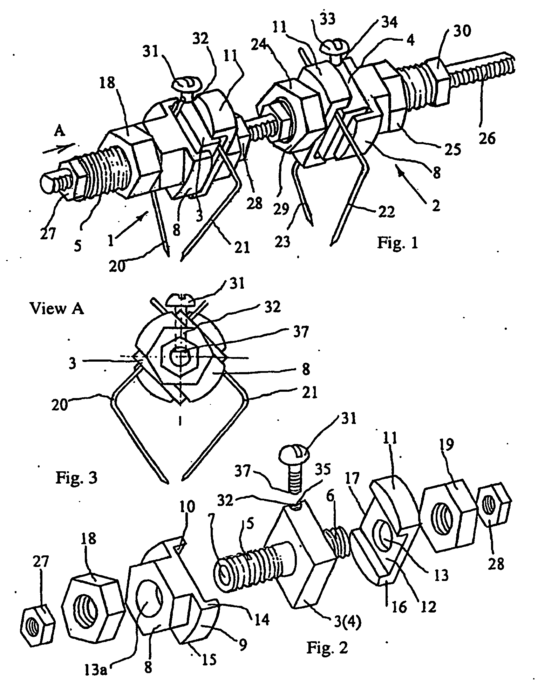

Referring to fig.1 a fixator for hand and foot bones is shown. A supporting element disassembled is shown according to the invention in general and fig.2.

Fixator of hand and foot bones according to the invention contains at least two supporting elements 1 and 2. Each of the supporting elements contains an settling bearer 3 or 4, each of them is made as a rectangular prism having settling cylinders 5 and 6 on both front surfaces. Each of the settling bearers has an adjusting through opening 7 (Fig.2). On the settling cylinder 5 there is a clamping element 8 made as a polyhedral prism having the base 9. The base is made as a cylinder cut longitudinally along two cords, in order to form two opposing surfaces and has an open longitudinal rectangular slot 10. An additional clamping element 11 is made as a cylinder cut off from opposite sides has an open longitudinal rectangular slot 12. The clamping element 8 and the additional clamping element 11 have through openings 13 and 13a respectively; the axes of the latter are parallel to the planes of cutting off, respectively 14, 15 and 16, 17. The clamping element 8 and the additional clamping element 11 are positioned, respectively, on settling cylinders 5 and 6, enclosing by longitudinal open rectangular slots 10 and 12, respectively, sides of each of the settling bearers 3 and 4. Ends of each of the wires 20 and 21, positioned at an angle, are attached by the clamping element 8 and the additional clamping element 11 with the help of fastening nuts 18 and 19 (Fig. 3). Ends of wires 22 and 23 are fixed in the supporting element with the help of fastening nuts 24 and 25 in a similar manner (Fig. 1). Settling bearers 3 and 4 are positioned on the rod 26-with the possibility of longitudinal transport using a pair of regulating nuts 27, 28 and 29, 30 respectively. Each of the settling bearers 3 and 4 have a locking element, namely 31 and 33. Each of the latter is placed in an opening 32 and 34, respectively. Axes of the latter are perpendicular to the axis of the adjusting opening 7 of each of the mentioned adjusting elements 3 and 4. Each of the mentioned openings 32 and 34 are made in a lateral edge 35 of each of the locking elements 3 and 4 made as a rectangular prism.

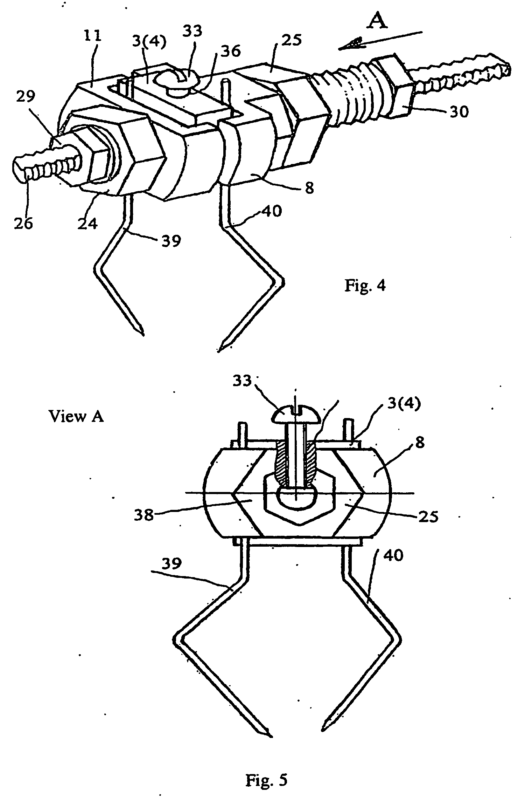

Besides, each of the settling bearers 3 and 4, made as a prism, has an opening 36 (Fig.4), made in one of its lateral sides. The axis of the opening is perpendicular to the axis of the mentioned longitudinal adjusting opening 7 of each of the adjusting elements 3 and 4.

Each of the locking elements 31 and 33 can be made as a screw (Fig. 1, 2, 3, 4, 5). Front surface 37 of each locking element (Fig. 3) and 38 (Fig. 5) is made perpendicular to its longitudinal axis.

Ends of wires 20 and 21 (Fig. 1, 2) can be curved at an angle to each other, as a variant.

Besides, each of the attached ends of wires 39 and 40 (Fig. 4, 5) are parallel to each other and the ends plunged into bone are at an angle to each other to have simple and reliable fixation.

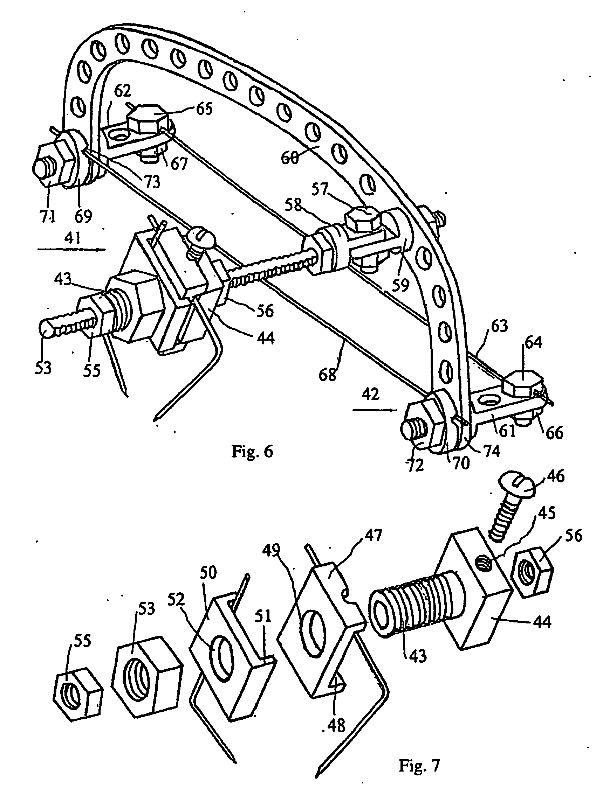

Fixator of hand and foot bones contains two supporting elements 41 and 42 (Fig. 6) for increasing the range of application. The first supporting element 41 (Fig. 7) include settling bearer made as a bushing 43 with a head 44. There is an opening 45 in the mentioned head 44. The axis of the opening is perpendicular to the axis of the bushing 43. There is a locking element 46 in the opening 45. The locking element is made as a screw; its front surface is perpendicular to the axis of the locking element 46. On external surface of the bushing 43 there is a threading; there is a clamping element 47 made as a prism with an open rectangular slot 48 and a through longitudinal adjusting opening 49. Besides, there are an additional clamping element 50, made as a prism with an open slot 51 and a through longitudinal opening 52, and a fixing element. The first supporting element with the longitudinal flat spot is positioned with the possibility of longitudinal transport on the rod 54 (Fig. 6) using two regulating elements 55, 56. The rod 54 is connected by one of the ends with the possibility of angular turn using connecting unit made of brackets 58 and 59 interconnected by bolted joint 57. The bracket 58 is attached on the end of rod 54. The bracket 59 is attached on the settling bearer made as an arch 60. At each end of the latter there are brackets 61 and 62; ends of the wire 63 are attached to them with the help of wire-fixation bolts 64 and 65 and nuts 66 and 67. Besides, ends of the wire 68 are attached to the support 60 on the opposite side using brackets 61 and 62, washers 69 and 70 and nuts 71 and 72. Washers 69 and 70 have slots 73 and 74 to position ends of the wire 68 in them.

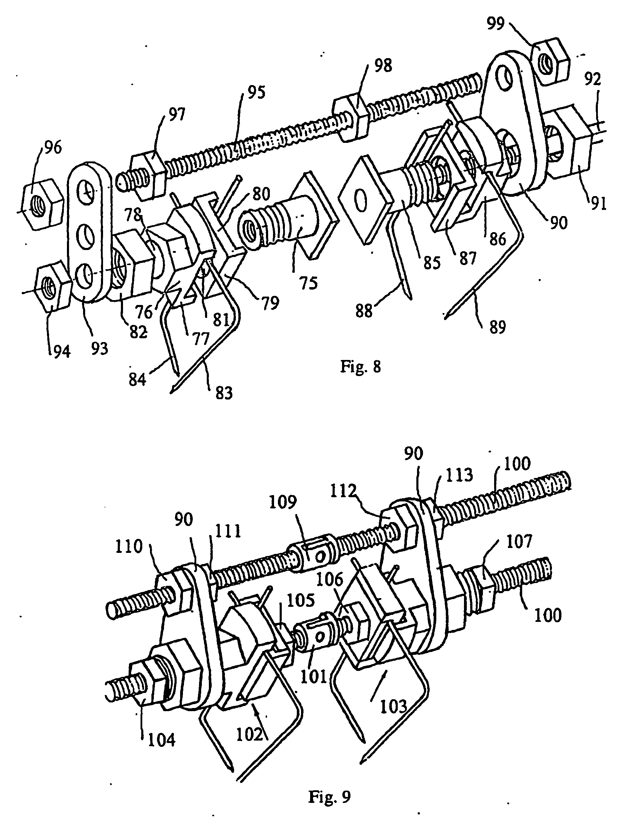

As a variant of the fixator for hand and foot bones, the first supporting element includes settling bearer 75 (Fig. 8) made as a bushing with a head. Threading is made on external and internal surfaces of the mentioned bushing. Head of the support 75 is made as a plate. The clamping element 76 is made as a prism with the base 77 and a through longitudinal adjusting opening 78. The additional clamping element 79 is made as a prism with an open slot 80 and a through longitudinal opening 81 to place it on the mounted support 75. The fixing element 82 is mounted on the mentionedsettling bearer 75. Ends of wires 83 and 84 are attached using fixing element 82. The second support element includes settling bearer 85 made as a bushing with the head. Internal surface of the mentioned bushing is smooth and the external one is threaded. The clamping element 86 has base and a through longitudinal adjusting opening. The additional clamping element 87 is made as a prism with an open slot and a through longitudinal adjusting opening.

Ends of wires 88 and 89 and a link 90 are mounted using tightening element 91.

Both the support elements 75 and 85 are connected with the help of the rod 92. One of its ends has a threading for mounting the support element 75, the connecting plate 93 and the regulating element 94. The support element 85 is mounted on the smooth end of the rod 92.

Connecting plate 93 and the link 90 have through openings with the help of which and an additional rod 95 and two pairs of regulating nuts 96, 97 and 98, 99 they are interconnected with the possibility of longitudinal transport.

The rod 100 is used for angular transport in a variant of invention. The rod has a hinge in the central part between two supporting elements 102 and 103. Both the supporting elements are made in a similar manner to the support element 85.

Each of the supporting elements 102 and 103 on the rod 100 is positioned with the possibility of transport using pairs of regulating nuts, respectively 104, 105 and 106, 107. Link 90 of each of the support elements is positioned with the possibility of longitudinal and angular transport on the additional rod 108. The latter had a hinge 109 in the central part. The link 90 of the supporting element 102 is placed on the additional rod 108 using additional pair of regulating nuts 110, 111; and the link 90 of the supporting element 103 is positioned on the additional rod 108 using an additional pair of the regulating nuts.

The present fixator of hand and foot bones is used the following way.

At least two pairs of wires 20, 21, 22, 23 are inserted into bone at an angle to each other (Fig. 1, 2, 3) distally and proximally to the supposed osteotomy site. Free ends of each pair of wires 20, 21 and 22, 23 are bent towards each other at approximately 90 degrees angle. Osteotomy is done after this. Then, the bent ends of each pair of wires 20, 21 and 22, 23 are positioned on both sides of the prism of the settling bearers 3, 4 and are attached using the clamping element 8, additional clamping element 11 and pairs of fastening nuts 18, 19 and 24, 25. Settling bearers 3, 4 are mounted on the rod 26 using pairs of nuts 27, 28 and 29, 30 and locking elements 31, 33 that are placed in openings 32, 34. The external surface of the rod 26 has Metric threading Distraction of bone fragments is done by transport of pairs of nuts 27, 28 and 29, 30 on the rod 26 to different sides. Locking screw 31 or 33 of the transported supporting element 3 and 4 is moved so as not to prevent transport and to prevent the turn of one or another locking element 3 or 4 at the expense of interaction of the frontal surface 37 or 38 of the locking screws 3, 4 with the flat spot on the rod 26.

One of the possible variants to fix a pair of wires 39, 40 (Fig. 4, 5) is a double bent of their ends. The ends, fixed by the clamping element 8 and the additional clamping element 11, are parallel. It allows redistributing the force applied during compression or distraction of bone fragments.

When lengthening metacarpals, two straight wires 68 and 63 fix their proximal fragments. The wires are attached to the ends of the arch 60 of the supporting element 42 (Fig. 6). The supporting element 41 is used for fixation of the distal fragment of the lengthened metacarpal; wires are positioned on one side from the head 44 of the bushing 43 between the clamping elements 47, 50 (Fig. 7). The head 44 is made as a tetrahedral prism; it enables holding the supporting element 41 with the wrench when attaching wires using the fixing element 53. Besides, locking element 46 is positioned in the opening 45, which is made in the side of the head 44; it prevents turn of the supporting element 41 relative to the rod 54. Interconnecting the supporting elements 41 and 42 is accomplished hingedly using the brackets 58, 59 and the bolted joint 57. Distraction is done with the help of transport of the supporting element 41 on the threaded rod 54 by unscrewing the nuts 55 and screwing the nut 56.

The fixator is mounted of two support elements for distracting when lengthening short fragments of small bones. One of the support elements contains the mounting support 75 (Fig. 8); ends of wires 83 and 84 are attached to it using clamping elements 76 and 79. Open slot 80 encloses the head of the mounting support 75. The base of the clamping element 76 encloses the clamping element 79. The mentioned clamping elements 76, 79 and ends of wires 83 and 84 are attached by the fixing nut 72. Threaded end of the rod 92 in screwed into the threaded opening of the mounting support 75. Connecting plate 93 is positioned on the same end and fixed using a nut 94. Settling bearer 85 is positioned on the smooth end of the rod 92; ends of wires 88 and 89 are attached to the support 85 using the link 90 and the fixing element 91. The connecting plate 93 and the link 90 are interconnected with the help of through openings by the additional rod 95 and two pairs of the regulating nuts, respectively 96, 97 and 98, 99. Distraction for lengthening of short bone fragments is done using transport of the settling bearer 85 with the help of regulating nuts 98, 99, unscrewing the regulating nut 99 and screwing the regulating nut 98 on the additional rod 95. The settling bearer 85 gets transported on the smooth part of the additional rod 92.

A variant of fixator for hand and foot bones having two rods 100 (Fig. 9) with articulated joins 109, 101 connecting the support elements 102, 103 is used for creating a deformity of a short bone or for management of articular pathology. The needed angular position is imparted to the bone fragments or finger phalanges by transporting nuts of a pair of the additional regulating nuts 110, 111 and 112, 113 and also a pair of the regulating nuts 104, 105 and 106, 107. Longitudinal transport of bone fragments is done if necessary using pairs of the regulating nuts 104, 105 and 106, 107.

### Industrial Applicability

The suggested fixator for hand and foot bones can be used for hand and foot bone lengthening, management of fractures of finger phalanges or metacarpals and metatarsals. The suggested fixator allows elimination of contractures of the adjacent joints, early full-value rehabilitation of hand and foot. Besides, the fixator can be effectively used for arthrodesis of interphalangeal and metacarpophalangeal joints. Construction of the patented fixator allows using it not only for 1, 5 metacarpals but also simultaneously for 2,3, 4 metacarpals, anomalies of hand development for lengthening of finger stumps. Assembling of the needed number of the support elements allows fixation of fragments of phalanx stump when lengthening the adjacent proximal short bone; hinge joints enable not only lengthening of phalanges but also simultaneous prevention of adjacent joints contractures. Application of the suggested fixator does not limit self-service possibilities of the patients, allows physical exercise therapy of hand for its functional restoration.

## Claims

1. A fixator for hand and foot bones comprising
a) at least two supporting elements (1, 2, 101, 102) each incorporating
- a settling bearer (3, 4, 75, 85) having a longitudinal adjusting through-opening (7),
- clamping elements (8, 11, 77, 86) with a longitudinal through-opening (13, 13a) and with a slot (10, 12) and
- fastening nuts (18, 19, 24, 25) pressing the clamping elements (8, 11, 77, 86) against the settling bearer (3, 4, 75, 85),
b) a threaded rod (26, 53, 92, 100) on which each of the supporting elements (1, 2) is mounted with a possibility of transport using two regulating nuts (27, 28, 29, 30), and
c) wires (20, 21, 22, 23) having one end for going through a bone and the other end for being attached to the supporting element (1, 2, 101, 102) in said slot (10,12) of the clamping elements
**characterized in that**
- one clamping element (8, 77, 86) is made in form of a polyhedral prism with a base (9),
- the settling bearer (3, 4, 75, 85) is made in the form of a rectangular prism provided on both frontal surfaces with settling cylinders (5, 6),
- the slot (10, 12) of the clamping elements (8, 11, 77, 86) is made in the form of an open longitudinal rectangular slot (10, 12) for enclosing the sides of the settling bearer (3, 4, 75, 85).

2. Fixator according to claim 1, **characterized in that** it is provided with an additional threaded rod (95) for shifting of
- a connecting plate (93) and
- a link (90), both fitted on one of the clamping element (86),
using regulating nuts (96, 97, 98, 99),
wherein the connecting plate (93) is mounted on the threaded rod (92),
wherein one end of the threaded rod (92) is smooth, and
wherein the longitudinal through-opening in one of the settling bearers (75) is threaded.

3. Fixator according to claim 1, **characterized by**
- an additional threaded rod (100),
- wherein the threaded rod (100) and the additional threaded rod (100) are provided with articulated joints (101, 109) located between the supporting elements (102, 103).

## Patentansprüche

1. Fixateur für Hand- und Fußknochen mit
a) mindestens zwei Halteelementen (1, 2, 101, 102), von denen jedes
- einen Einrichtträger (3, 4, 75, 85) mit einer längsverlaufenden durchgehenden Öffnung (7) für das Einstellen,
- Klemmelemente (8, 11, 77, 86) mit einer längsverlaufenden Durchgangsöffnung (13, 13a) und mit einem Schlitz (10, 12) und
- Befestigungsmuttern (18, 19, 24, 25) aufweist, die die Klemmelemente (8, 11, 77, 86) gegen den Einstellträger (3, 4, 75, 85) drücken,
b) einer Gewindestange (26, 53, 92, 100), an der jedes der Halteelemente (1, 2) mit einer Transportmöglichkeit unter Verwendung von zwei Reguliermuttern (27, 28, 29, 30) angebracht ist, und
c) Drähten (20, 21, 22, 23) deren eines Ende für den Durchgang durch einen Knochen und deren anderes Ende für die Befestigung an dem Halteelement (1, 2, 101, 102) in dem Schlitz (10, 12) der Klemmelemente vorgesehen ist,
**dadurch gekennzeichnet,**
- **dass** ein Klemmelement (8, 77, 86) in Form eines Polyederprismas mit einer Basis (9) ausgebildet ist,
- **dass** der Einrichtträger (3, 4, 75, 85) in Form eines Rechteckprismas ausgebildet ist, das an beiden Frontflächen mit Einrichtzylindern (5, 6) versehen ist, und
- **dass** der Schlitz (10, 12) der Klemmelemente (8, 11, 77, 86) in Form eines offenen längsverlaufenden rechteckigen Schlitzes (10, 12) zum Umschließen der Seiten des Einrichtträgers (3, 4, 75, 85) ausgebildet ist.

2. Fixateur nach Anspruch 1, **dadurch gekennzeichnet,**
- **dass** er mit einer zusätzlichen Gewindestange (95) zum Verschieben einer Verbindungsplatte (93) und eines Gliedes (90), die beide an einem der Klemmelemente (86) befestigt sind, unter Verwendung der Reguliermuttern (96, 97, 98, 99) versehen ist,
- wobei die Verbindungsplatte (93) an der Gewindestange (92) angebracht ist,
- wobei ein Ende der Gewindestange (95) glatt ist, und
- wobei die längsverlaufende durchgehende Öffnung in einem der Einrichtträger (75) mit Gewinde versehen ist.

3. Fixateur nach Anspruch 1, **gekennzeichnet durch** eine zusätzliche Gewindestange (100), wobei die Gewindestange (100) und die zusätzliche Gewindestange (100) mit Gelenkverbindungen (101, 109) versehen sind, die zwischen den Halteelementen (102, 103) angeordnet sind.

## Revendications

1. Fixateur pour os de la main et du pied comprenant
a) au moins deux éléments de support (1, 2, 101, 102) incorporant chacun
- un support de maintien (3, 4, 75, 85) ayant une ouverture traversante d'ajustement longitudinale (7),
- des éléments d'immobilisation (8, 11, 77, 86) avec une ouverture traversante longitudinale (13, 13a) et avec une fente (10, 12) et
- des écrous de serrage (18, 19, 24, 25) pressant les éléments d'immobilisation (8, 11, 77, 86) contre le support de maintien (3, 4, 75, 85),
b) une tige filetée (26, 53, 92, 100) sur laquelle chacun des éléments de support (1, 2) est monté avec une possibilité de transport en utilisant deux écrous de régulation (27, 28, 29, 30), et
c) des fils (20, 21, 22, 23) ayant une extrémité pour passer à travers un os et l'autre extrémité pour être attachée à l'élément de support (1, 2, 101, 102) dans ladite fente (10, 12) des éléments d'immobilisation
**caractérisé en ce que**
- un élément d'immobilisation (8, 77, 86) est fait sous la forme d'un prisme polyédrique avec une base (9),
- le support de maintien (3, 4, 75, 85) est fait sous la forme d'un prisme rectangle prévu sur les deux surfaces frontales avec des cylindres de maintien (5, 6),
- la fente (10, 12) des éléments d'immobilisation (8, 11, 77, 86) est faite sous la forme d'une fente rectangulaire longitudinale ouverte (10, 12) pour renfermer les côtés du support de maintien (3, 4, 75, 85).

2. Fixateur selon la revendication 1, **caractérisé en ce qu'**il est prévu avec une tige filetée additionnelle (95) pour le déplacement de
- une plaque de connexion (93) et
- une liaison (90), les deux disposées sur l'un de l'élément d'immobilisation (86),
en utilisant des écrous de régulation (96, 97, 98, 99),
dans lequel la plaque de connexion (93) est montée sur la tige filetée (92),
dans lequel une extrémité de la tige filetée (92) est lisse, et
dans lequel l'ouverture traversante longitudinale dans l'un des supports de maintien (75) est filetée.

3. Fixateur selon la revendication 1, **caractérisé par**
- une tige filetée additionnelle (100),
- dans lequel la tige filetée (100) et la tige filetée additionnelle (100) sont prévues avec des joints articulés (101, 109) situés entre les éléments de support (102, 103).
